# EUROPEAN PATENT APPLICATION

(11) **EP 4 339 885 A1**
(43) Date of publication of application: **20.03.2024**
(21) Application number: 22807122.1
(22) Date of filing: 04.03.2022
(51) Int. Cl.: G06T 7/00, G06T 7/70, A61B 5/107, A61B 5/11

(54) **SLEEP-PERIOD MOVING IMAGE ANALYSIS METHOD AND SLEEP-PERIOD MOVING IMAGE ANALYSIS DEVICE**

(30) Priority: 13.05.2021 JP 2021081445
(71) Applicant: Shimadzu Corporation, Nakagyo-ku, Kyoto-shi, Kyoto 604-8511 (JP)
(72) Inventor: AKAZAWA, Ayako, Kyoto-shi, Kyoto 604-8511 (JP); NAGASHIMA, Tomotaka, Kyoto-shi, Kyoto 604-8511 (JP); SOTOGUCHI, Akie, Kyoto-shi, Kyoto 604-8511 (JP)
(74) Representative: Kilian Kilian & Partner mbB
(86) International application number: PCT/JP2022/009521
(87) International publication number: WO 2022/239415

(57) **Abstract**

A sleeping video analysis method according to this invention includes a lying posture estimation step (step 311) of estimating a lying posture of a subject (200) while sleeping based on a sleeping video of a subject (200) while sleeping; a lying posture classification step (step 313) of classifying the lying posture based on a lying posture estimation result(s) of the lying posture estimation step (step 311); and a body movement measurement step (step 314) of measuring body movement based on the lying posture estimation result(s) of the lying posture estimation step (311). The sleeping video analysis method includes a display step of displaying at least one of a lying posture classification result(s) and a body movement measurement result(s).

## Description

### Technical Field

The present invention relates to a sleeping video analysis method and a sleeping video analysis apparatus.

### Background Art

Conventionally, a posture estimation method (video analysis method) which captures images (video) of a person and estimates a posture of the person based on the captured video is known. Such a posture estimation method (video analysis method) is disclosed in Japanese Patent Laid-Open Publication No. JP 2019-219989, for example.

The above Japanese Patent Laid-Open Publication No. JP 2019-219989 discloses a posture estimation method (video analysis method) of estimating a posture of a person based on subject images of the person. In the posture estimation method disclosed in the above Japanese Patent Laid-Open Publication No. JP 2019-219989, a rotated image is generated by rotating the subject image to an orientation in which a posture of the person can be easily estimated. Subsequently, the posture of the person is estimated based on the rotated image in which the subject image is rotated, and posture estimation results representing the positions of joint points of the person are generated.

### Prior Art

### Patent Document

Patent Document 1: Japanese Patent Laid-Open Publication No. JP 2019-219989

### Summary of the Invention

### Problems to be Solved by the Invention

For purposes of treatment for or investigation of sleep disorders, etc., users such as physicians sometimes necessarily capture video of persons (subjects) while sleeping and see their lying postures of the subjects while sleeping (sleeping postures), such as supine, prone and lateral decubitus positions, in the video captured while sleeping (sleeping videos). In the case in which users necessarily see lying postures as discussed above, the users are required to determine lying posture changes of a subject in sleep which is long time by themselves. For this reason, there is a problem that users cannot easily grasp the lying posture changes of a subject in the sleeping video.

The present invention is intended to solve the above problem, and one object of the present invention is to provide a sleeping video analysis method and a sleeping video analysis apparatus capable of allowing users to easily grasp lying posture changes of a subject in a sleeping video.

### Means for Solving the Problems

A sleeping video analysis method according to a first aspect of the present invention includes a lying posture estimation step of estimating a lying posture of a subject while sleeping based on a sleeping video of the subject while sleeping; a lying posture classification step of classifying the lying posture of the subject while sleeping based on a lying posture estimation result(s) of the lying posture estimation step; a body movement measurement step of measuring body movement of the subject while sleeping based on the lying posture estimation result(s) of the lying posture estimation step; and a display step of displaying at least one of a lying posture classification result(s) of the lying posture classification step and a body movement measurement result(s) of the body movement measurement step.

A sleeping video analysis apparatus according to a second aspect of the present invention includes a controller configured to analyze a sleeping video of a subject while sleeping; and a display configured to display an analysis result(s) analyzed by the controller, wherein the controller is configured to control a lying posture estimation function of estimating a lying posture of the subject while sleeping based on the sleeping video, a lying posture classification function of classifying the lying posture of the subject while sleeping based on a lying posture estimation result(s) in the lying posture estimation function, a body movement measurement function of measuring body movement of the subject while sleeping based on the lying posture estimation result(s) of the lying posture estimation function, and a display function of displaying at least one of a lying posture classification result(s) of the lying posture classification function and a body movement measurement result(s) of the body movement measurement function.

### Effect of the Invention

According to the present invention, because the lying posture classification result(s) classified based on the lying posture estimation result(s) is/are displayed, users can see the lying posture classification result(s) and know the classification of the lying posture of a subject while sleeping. As a result, dissimilar to a case in which a lying posture classification result(s) is/are not displayed, users can easily grasp the lying posture classification and lying posture changes of the subject in a sleeping video. Also, a body movement measurement result(s) measured based on a lying posture estimation result is/are displayed, users can see the body movement measurement result(s) and know an amount of body movement change of the subject while sleeping. As a result, dissimilar to a case in which a body movement measurement result(s) is/are not displayed, users can easily grasp body movement changes of the subject in a sleeping video. Therefore, users can easily grasp lying posture changes (sleeping posture changes) of a subject in a sleeping video from at least one of the lying posture classification result(s) and the body movement measurement result(s) .

### Brief Description of the Drawings

[FIG. 1] Block diagram showing the overall configuration of a sleeping video analysis apparatus according to one embodiment of the present invention.
[FIG. 2] First diagram illustrating selection of a frame from a sleeping video.
[FIG. 3] Second diagram illustrating selection of frames from the sleeping video.
[FIG. 4] Third diagram illustrating selection of frames from the sleeping video.
[FIG. 5] Fourth diagram illustrating selection of frames from the sleeping video.
[FIG. 6] Fifth diagram illustrating selection of frames from the sleeping video.
[FIG. 7] Diagram showing generation of a shortened video from the sleeping video.
[FIG. 8] Diagram illustrating rotation angle determination.
[FIG. 9] Diagram showing a flow of acquisition of lying posture classification results and body movement measurement results from lying posture estimation.
[FIG. 10] Diagram showing an exemplary indication on a display.
[FIG. 11] Diagram showing another exemplary indication on the display.
[FIG. 12] First flowchart showing control by a PC in sleeping video analysis.
[FIG. 13] Second flowchart showing control by a PC in sleeping video analysis.
[FIG. 14] Diagram showing an exemplary indication on a display according to a first modified example.
[FIG. 15] Diagram showing exemplary processing for rotation angle determination according to a second modified example.

### Modes for Carrying Out the Invention

Embodiments embodying the present invention are hereinafter described on the basis of the drawings.

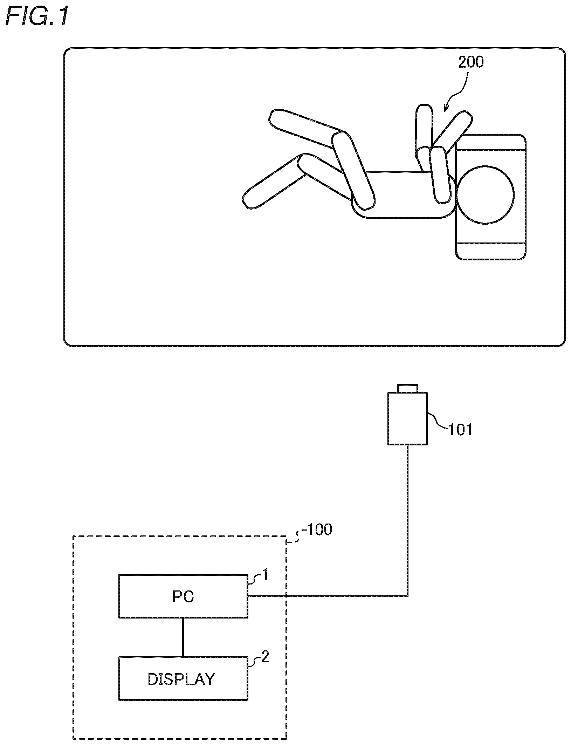

As shown in FIG. 1, an analysis apparatus 100 includes a PC 1 and a display 2. The analysis apparatus 100 is configured to analyze videos (sleeping videos) of subjects 200 captured by a camera 101 while sleeping. For example, the camera 101 is a night vision cameras configured to capture images in the dark. The camera 101 is not necessarily constantly connected to the PC 1. For example, a video (sleeping video) of a subject 200 captured while sleeping can be saved to an internal or external recording medium of the camera 101 so that the PC 1 can analyze the stored sleeping video at a later date (after the image capture). The analysis apparatus 100 is an example of a "sleeping video analysis apparatus" in the claims.

The subject 200 is a child (small child), for example. Because the analysis apparatus 100 (sleeping video analysis method) can analyze movement of a body (body movement) of the subject 200 while sleeping, the movement of the body (body movement) can be analyzed without sensors such as acceleration sensors or position sensors attached to the body (non-contact analysis). Accordingly, even in a case in which a child (small child) is unwilling to attach sensors on his/her body or detaches sensors attached to his/her body, movement of the body (body movement) of the child (small child) during sleep can be easily analyzed. The subject 200 is not limited to a child.

The PC 1 is a personal computer including a CPU (Central Processing Unit), GPU (Graphics Processing Unit), ROM (Read Only Memory) and RAM (Random Access Memory), etc. Also, the PC 1 includes a storage 22 such as HDD (Hard Disk Drive) and SSD (Solid State Drive). The PC 1 is configured to analyze a sleeping video, which is a moving image of the subject 200 while sleeping. The PC 1 is configured to take moving image data of the sleeping video captured by the camera 101. The PC 1 is an example of a "controller" in the claims.

Also, the PC 1 records (stores) a program that executes the PC 1 to perform the sleeping video analysis method (control of functions of analyzing a sleeping video) described later. The program, which executes the analysis apparatus 100 (PC 1) to perform the sleeping video analysis method can be stored in a server on the network.

For example, the display 2 is a liquid crystal display or an organic electroluminescence display. For example, the display 2 is connected to the PC 1 through an audio and video interface, such as HDMI (registered trademark). The display 2 is an example of a "display" in the claims.

### (Selection Control)

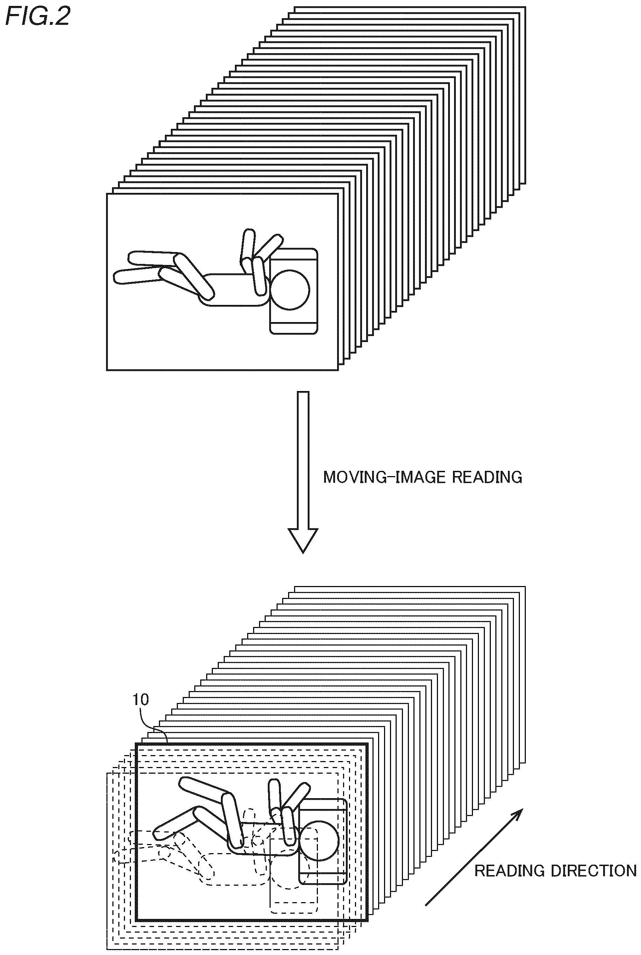

The PC 1 is configured to control a function of sequentially reading images of frames in the sleeping video, and selecting frames that have a difference between images of frames not smaller than a selection threshold in the sleeping video as selected frames corresponding to a change of lying posture of the subject (positions of joint points of the subject 200) in the sleeping video (selection control). Control of a frame selection (frame pickup) function by the PC 1 is now described with reference to FIG. 2 to 6.

In this embodiment, the PC 1 sequentially reads images of frames in the sleeping video, and can select a frame that has a difference between images of frames not smaller than a selection threshold in the sleeping video as a selected frame corresponding to a lying posture change of the subject 200 in the sleeping video. Also, the PC 1 additionally selects frames previous and subsequent to the selected frame as frames that include images to be used to estimate a lying posture (to estimate joint points) in addition to the selected frame.

Specifically, the PC 1 sequentially reads images of frames in the sleeping video and selects a frame that has a difference between images of frames adjacent to each other not smaller than a first selection threshold in the sleeping video as a first selected frame 10 (see FIG. 2).

Techniques that acquire pixel differences and a correlation between images can be used to calculate the difference between the images. The difference between the images is represented by a scalar value, and the PC 1 selects a frame that has a value falling within a predetermined range as a frame of an image to be used to estimate a lying posture. For example, ZNCC (Zero-Mean Normalized Cross Correlation) is used as a technique that calculates the difference between frames in the sleeping video. ZNCC (Zero-Mean Normalized Cross Correlation) calculates a correlation between images in frames. Calculated values in ZNCC (Zero-Mean Normalized Cross Correlation) range from -1.0 to 1.0, and the lower the calculated value, the lower the matching degree (the greater the difference).

In a case in which ZNCC (Zero-Mean Normalized Cross Correlation) is used as a technique that acquires pixel differences and a correlation between images, a threshold is set for calculated values (matching degrees) between frames adjacent to each other in the sleeping video so that a frame that is currently read (current frame) is selected as the selected frame (first selected frame 10) if its calculated value (its matching degree) is not lower than the threshold (if its difference between an image of the current frame and an image of a frame immediately previous to the current frame is not smaller than the first selection threshold).

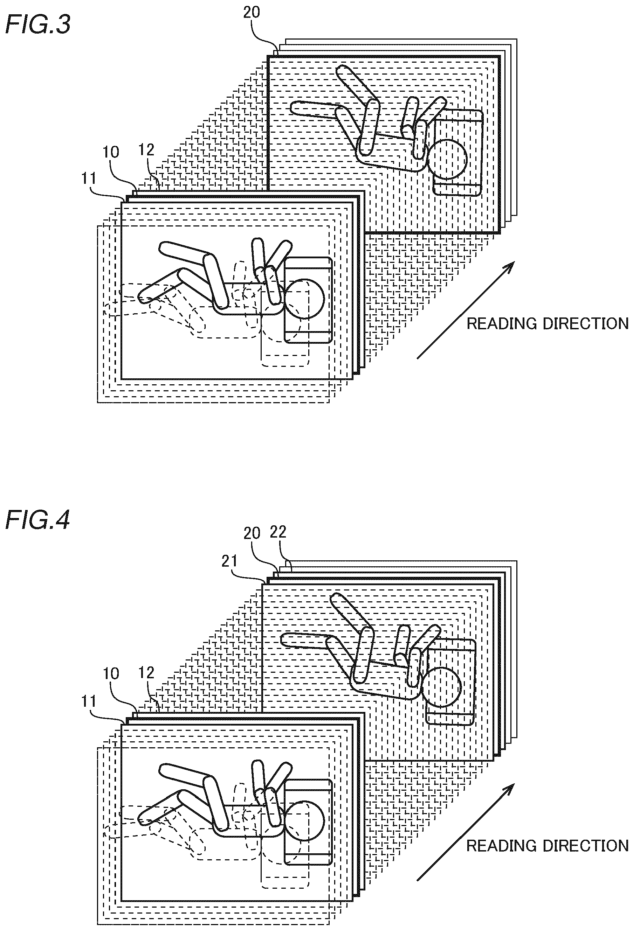

Also, as shown in FIG. 3, the PC 1 additionally selects frames (frames 11 and 12) previous and subsequent to the first selected frame 10 as frames that include images to be used to estimate a lying posture in the lying posture estimation in addition to the first selected frame 10.

Also, as shown in FIG. 3, the PC 1 is configured to select a frame that follows to the first selected frame 10 and has a difference between images of this frame and the first selected frame 10 that is followed by and the closest to this frame not smaller than a second selection threshold in the sleeping video as a second selected frame 20. The second selection threshold value can be the same value as or a value different from the first selection threshold.

Also, as shown in FIG. 4, the PC 1 additionally selects frames (frames 21 and 22) previous and subsequent to the second selected frame 20 as frames that include images to be used to estimate a lying posture in the lying posture estimation in addition to the second selected frame 20.

Also, the PC 1 is configured to select frames that are spaced at a predetermined interval from each other from the first selected frame 10 that is followed by and the closest to the second selected frame 20 to the second selected frame 20 as frames that include images to be used to estimate a lying posture in the lying posture estimation in addition to the first and second selected frames 10 and 20.

Specifically, as shown in FIG. 4, the PC 1 is configured to select frames that are spaced at a predetermined interval from each other from the first selected frame 10 that is followed by and the closest to the second selected frame 20 to the second selected frame 20 as frames (frames 30, 40 and 50) that include images to be used to estimate a lying posture.

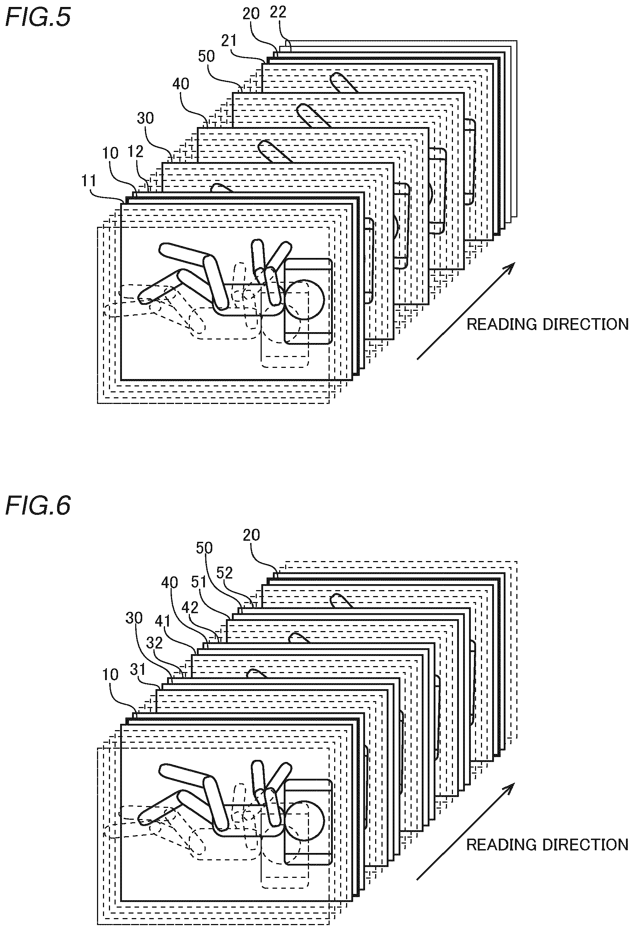

Also, as shown in FIG. 6, the PC 1 additionally selects frames (frames 31 and 32) previous and subsequent to the second selected frame 30, frames (frames 41 and 42) previous and subsequent to the second selected frame 40, and frames (frames 51 and 52) previous and subsequent to the second selected frame 50 as frames that include images to be used to estimate a lying posture in the lying posture estimation.

In the above description and FIGS. 3 to 6, for sake of illustration, one frame is illustratively selected as each of previous and subsequent frames, which are previous and subsequent to each of the first and second selected frames 10 and 20, and the frames 30, 40 and 50. However, note that, as previous or subsequent frames of each of the first and second selected frames 10 and 20, and the frames 30, 40 and 50, one or more frames (a certain number of frames providing a margin on previous side or subsequent side of the frame) are selected in actual selection of the previous or subsequent frames.

In other words, when previous and subsequent frames of each of the first and second selected frames 10 and 20, and the frames 30, 40 and 50 are selected as frames to be used to estimate a lying posture in the lying posture estimation, frames selected as the previous and subsequent frames are not limited to frames immediately previous and subsequent to the frame.

Specifically, the PC 1 can select a certain number of frames or frames in a certain time period adjacent to each of the first and second selected frames 10 and 20, and the frames 30, 40 and 50 as frames that include images to be used to estimate a lying posture in the lying posture estimation. For example, the PC 1 selects frames in several seconds previous to and frames in several seconds subsequent to each of the first and second selected frames 10 and 20, and the frames 30, 40 and 50 as frames that include images to be used to estimate a lying posture in the lying posture estimation in addition to the first and second selected frames 10 and 20, and the frames 30, 40 and 50.

### (Control of Video-Shortening Control)

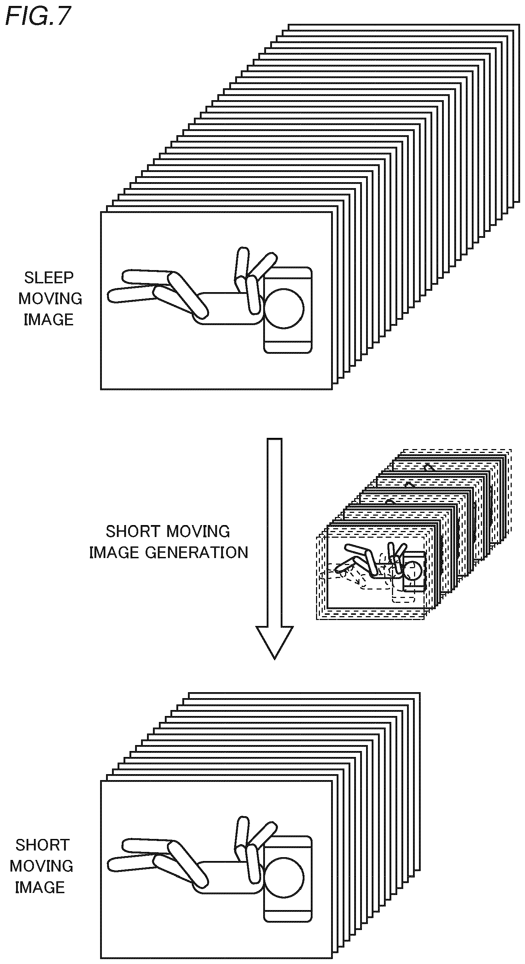

As shown in FIG. 7, the PC 1 is configured to control a function of combining the selected frames selected from the sleeping video in the selection function so as to generate and provide a shortened video (control of Video-shortening control).

Specifically, the PC 1 is configured to use, in addition to a plurality of selected frames (a plurality of first selected frames 10 and a plurality of second selected frames 20), frames (frames 30, 40 and 50) that are spaced at a predetermined interval from each other which are selected from the sleeping video as frames from the first selected frame 10 to the second selected frame 20 in the generation of the shortened video. Also, the PC 1 uses, in addition to the first and second selected frames 10 and 20, and the frames 30, 40 and 50, images of previous and subsequent frames of the first and second selected frames 10 and 20, and the frames 30, 40 and 50 in the generation of the shortened video. Here, FPS (Frames Per Second) of the shortened video is unchanged from the video before shortening. The PC 1 can temporarily or permanently hold parts (frame number or time range) of the sleeping video (original moving image) corresponding to the selected frames, and record (store) and provide correspondence of frames of the shortened video to the sleeping video (original video).

### (Rotation Angle Determination Control)

The PC 1 can control a function of rotating images of frames in the shortened video by a plurality of angles and selectively determining an image rotation angle to be used to estimate the lying posture in the lying posture estimation from the images rotated at the timing of at least one of every time a predetermined time elapses in the shortened video and every time a difference between images of one frame and a previous frame in the shortened video becomes not smaller than a rotation threshold (rotation angle determination control).

In this embodiment, the PC 1 carries out (controls) a function of sequentially reading images of frames in the shortened video, a function of rotating images of frames in the shortened video by a plurality of angles, and a function of selectively determining an image rotation angle to be used to estimate the lying posture in the lying posture estimation from the images rotated at the timing of every time the predetermined time elapses in the shortened video and every time a difference between images of one frame and a previous frame in the shortened video becomes not smaller than a rotation threshold. In other words, the PC 1 sequentially reads images of frames in the shortened video, and selectively determines an image rotation angle to be used to estimate the lying posture in the lying posture estimation from the images rotated prior to the lying posture estimation if any of predetermined conditions is satisfied in an image in the read frames (every time the predetermined time elapses in the shortened video, or every time a difference between images of one frame and a later frame in the shortened video becomes not smaller than a rotation threshold).

If none of the predetermined conditions is satisfied in an image of the read frame, the PC 1 does not perform the rotation angle determination control, and rotates, prior to the lying posture estimation, the image to the same rotation angle as a frame that is the closest to this frame and has been determined its rotation angle. In other words, in the sequential reading of images of frames in the shortened video, an image rotation angle to be used to estimate the lying posture in the lying posture estimation from the images rotated is changed (selectively determined) only if predetermined conditions are satisfied (every time the predetermined time elapses in the shortened video, or every time a difference between images of one frame and a later frame in the shortened video becomes not smaller than a rotation threshold).

In this embodiment, the PC 1 can rotate images of frames in the shortened video by a plurality of angles every time the predetermined time elapses in the shortened video, or every time a difference between images of one frame and a later frame in the shortened video becomes not smaller than a rotation threshold, and estimate a lying posture of the subject 200 while sleeping for each of the images rotated to a plurality of angles. An image rotation angle to be used to estimate the lying posture in the lying posture estimation is determined by using a technique similar to the later-discussed lying posture estimation (lying posture estimation model).

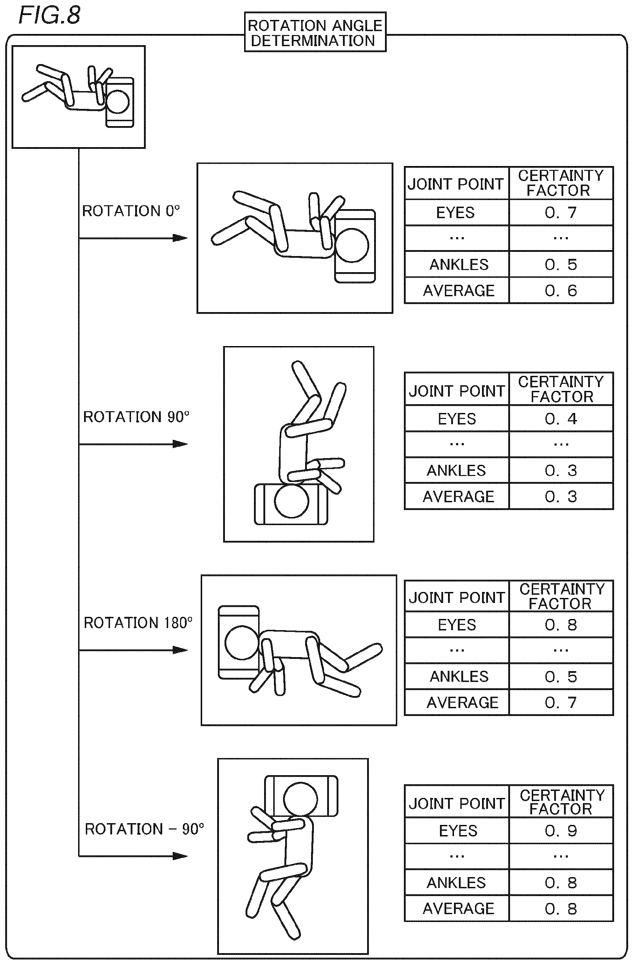

Specifically, the PC 1 receives images that have been rotated to rotation angles as inputs, and determines, as the rotation angle, an image rotation angle that can provide optimal accuracy of lying posture estimation based on certainty factors of joint points provided by the lying posture estimation model, which is a learned model configured to provide joint points and certainty factors as discussed later. For example, as shown in FIG. 8, from images rotated by a plurality of angles (0°, 90°, 180°, and -90°) in each frame in the shortened video, a rotation angle of the image that provides the highest average value of the certainty factors of joint points provided by the lying posture estimation model is selected as the image rotation angle that can provide optimal accuracy of lying posture estimation so that it is determined as rotation angle to which the image is changed. In a case of FIG. 8, a rotation angle -90° which provides the highest average value of the certainty factors is determined as the image rotation angle. The plurality of angles by which an image is rotated to determine the image rotation angle can be incremented by 45°, etc.

### (Lying Posture Estimation Control)

The PC 1 is configured to control a function of estimating a lying posture of the subject 200 while sleeping based on the sleeping video (lying posture estimation control). The PC 1 can estimate a lying posture of the subject 200 while sleeping based on the learned model generated by machine learning.

In this embodiment, the PC 1 estimates a lying posture of the subject while sleeping by using an image of a selected frame, which is selected in the selection control. The PC 1 estimates a lying posture of the subject 200 while sleeping by using a learned model produced by machine learning (lying posture estimation model).

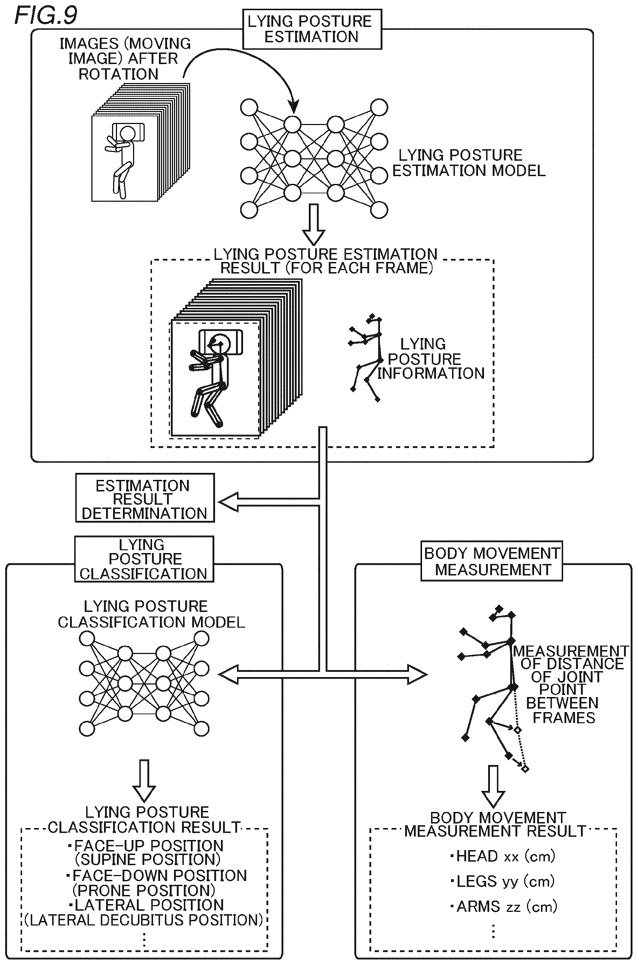

Specifically, the PC 1 uses the lying posture estimation model that provides joint points and certainty factors as output. For example, the PC 1 uses deep learning models such as Higher HRNet, OpenPose as the lying posture estimation model. As shown in Figure 9, the lying posture estimation model receives a rotated image of each frame in the shortened video as input, and provides positions of joint points of the subject 200 and certainty factors as output. The definition of joint points depends on data used to learn the model. For example, the lying posture estimation model provides position of eyes, nose, ears, shoulders, elbows, wrists, waist, knees, ankles, etc. as output.

Because the lying posture estimation result will include noise (even if the subject 200 is not in motion, the result may vary due to influences of noise, etc.), noise removal processing can be applied to the lying posture estimation result.

### (Estimation Result Determination Control)

If lying posture estimation has a low degree of accuracy, such low accuracy will affect the later-discussed lying posture classification and body movement measurement. For this reason, to prevent output of low accurate lying posture classification and body movement measurement due to input of low accurate lying posture estimation result, a provided lying posture estimation result (see FIG. 9) is determined whether the lying posture estimation result is available (whether the lying posture estimation result is valid).

In this embodiment, the PC 1 is configured to determine based on a certainty factor of the lying posture estimation acquired by the lying posture estimation model (learned model) in the lying posture estimation in the sleep of the subject 200 and a predetermined validity threshold whether lying posture estimation results estimated by the lying posture estimation model are valid.

Specifically, a threshold (validity threshold) is specified corresponding to a certainty factor of a position of each joint point, which is output of the lying posture estimation model (lying posture estimation result), so that it is determined whether lying posture classification and body movement measurement can be validly performed based on the lying posture estimation result (position of each joint point) as output.

### (Lying Posture Classification Control)

The PC 1 is configured to control a function of classifying the lying posture of the subject 200 while sleeping based on lying posture estimation results in the lying posture estimation control (lying posture classification control).

The PC 1 can receive a lying posture estimation result as input, and provide classification of the lying posture of the subject 200 while sleeping such as face-up position (supine position), face-down position (prone position) and lateral position (lateral decubitus position) as output (see FIG. 9) in the lying posture classification control.

Specifically, the PC 1 uses a lying posture classification model produced to provide classification of the lying posture of the subject 200 while sleeping such as face-up position (supine position), face-down position (prone position) and lateral position (lateral decubitus position) as output. For example, the PC 1 uses a deep learning model such as DNN (Deep Neural Network) or GCN (Graph Convolutional Network) as the lying posture classification model. In this embodiment, the lying posture classification model can receive a lying posture estimation result (positions of joint points) as input, and provide classification of the lying posture of the subject 200 while sleeping such as face-up position (supine position), face-down position (prone position) and lateral position (lateral decubitus position) as output (see FIG. 9).

Instead of deep learning models, SVM (Support Vector Machine) or logistics regression can be used for lying posture classification. In addition to the lying posture classification receiving the lying posture estimation results as input, lying posture classification that directly receives images in the shortened video or sleeping video and classifies a lying posture of the subject by using a deep learning model such as CNN (Convolutional Neural Network).

Note that, in the present embodiment, the PC 1 performs the classification of a lying posture of subject 200 if it is determined that a lying posture estimation result in the lying posture estimation is valid in the estimation result determination.

### (Body Movement Measurement Control)

The PC 1 is configured to control a function of measuring body movement of the subject 200 while sleeping based on lying posture estimation results in the lying posture estimation control (body movement measurement control). As shown in FIG. 9, the PC 1 provides a difference between the current frame and an immediately previous frame (or a previous frame at a predetermined number of shots before the current frame or specified by some conditions) as output of body movement (body movement measurement).

In the body movement measurement control, as shown in FIG. 9, the difference of the lying posture estimation results (positions of each joint point) of frames in the shortened video is provided for joint points (eyes, nose, ears, shoulders, elbows, wrists, waist, knees, ankles, etc.) in each frame in the shortened video as body movement (body movement measurement result).

### (Display Control)

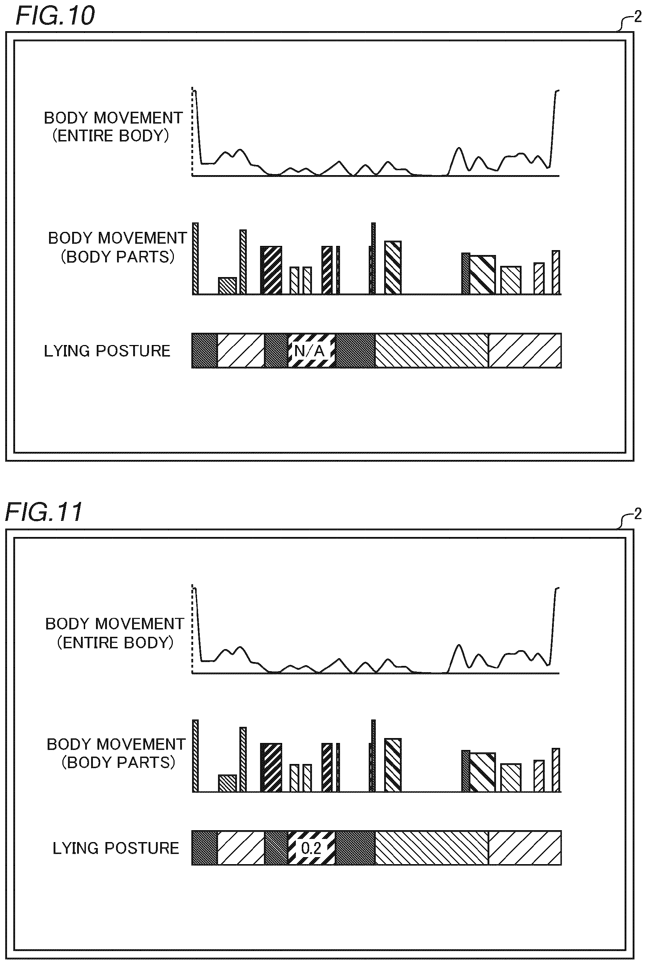

The PC 1 is configured to control a display function of displaying at least one of lying posture classification results in the lying posture classification control and body movement measurement results in the body movement measurement control. In this embodiment, as shown in FIG. 10, the display 2 is configured to display both the lying posture classification results and the body movement measurement results based on control by the PC 1.

The display 2 can display the lying posture classification results and the body movement measurement results in a time series based on control by the PC 1, as shown in FIG. 10. The lying posture classification results are displayed by a component bar chart representing lying posture variation by applying different colors to different classified lying postures. The body movement measurement results are indicated separately for body movement of the entire body and body movement of parts (head, arms, legs, etc.). Parts can be selectively indicated, for example only arms or legs can be selected to be indicated in accordance with user request (information required by a user). The body movement results of the parts are indicated by different colors assigned to the parts so that body movement changes of the parts can be recognized. In addition, the display 2 can display a graph that indicates the body movement changes of the parts in a time series. The body movement results as output can be represented by continuous values or discrete values in accordance with magnitude of body movement.

The display 2 displays the lying posture classification results in case in which lying postures are classified in the lying posture classification in accordance with determination results of the estimated result determination. On the other hand, if a lying posture is not classified in the lying posture classification in accordance with a determination result of the estimated result determination, an indication that indicates that the lying posture is not classified (see FIG. 10). In exemplary display control, an indication such as "N/A" or No Determination can be indicated on the component bar chart as shown in FIG. 10.

In this embodiment, the analysis apparatus 100 is configured to switch the indication to an indication indicating a certainty factor of lying posture estimation estimated by using the lying posture estimation model (see FIG. 11) if a lying posture is not classified in the lying posture classification in accordance with a determination result of the estimated result determination. For example, when the indication is switched, a value "0.2" that is not greater than a predetermined threshold (validity threshold) is indicated on the component bar chart as shown in FIG. 11.

### (Sleeping video Analysis)

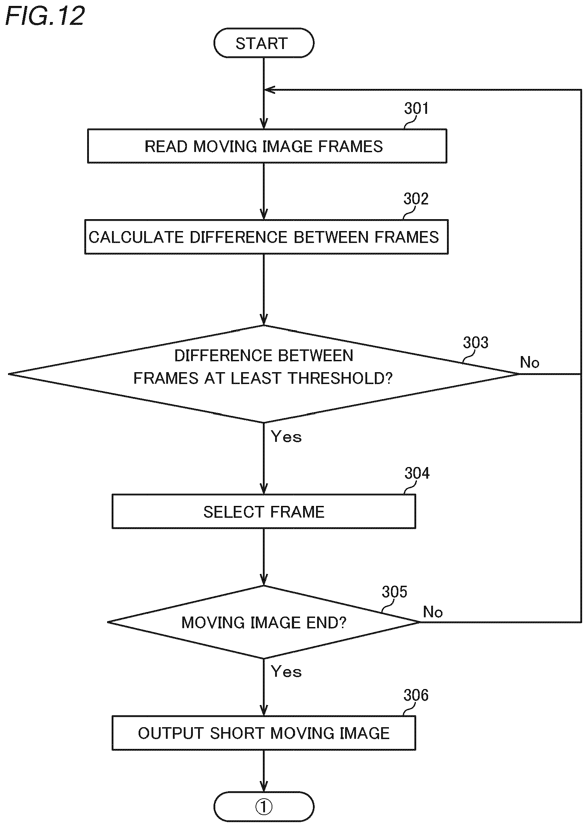

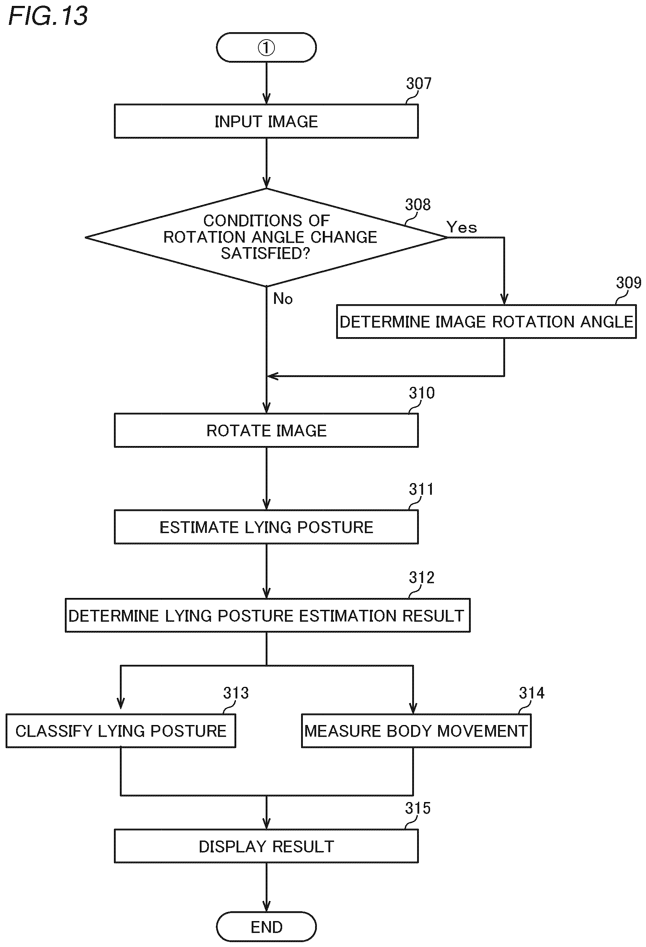

A processing flow of the sleeping video analysis executed by the analysis apparatus 100 (PC 1) according to the present embodiment is now described with reference to FIGS. 12 and 13.

As shown in FIG. 12, moving image frames in the sleeping video are first read (reading of moving image frames) in step 301. Subsequently, the procedure goes to step 302.

In step 302, a difference between the frames is calculated. In step 302, the difference between frames of a sleeping video that are sequentially read is calculated using the aforementioned technique (ZNCC: Zero mean normalized cross-correlation). Subsequently, the procedure goes to step 303.

In step 303, it is determined whether the difference between frames is at least a threshold. As discussed above, if it is determined that the difference between frames is the threshold or greater (at least a first or second selection threshold), the procedure goes to step 304. If it is not determined that the difference between frames is the threshold or greater (at least a first or second selection threshold), the procedure goes back to step 301.

In step 304, frame selection is performed. Specifically, the frame that has a difference between images of frames adjacent to each other not smaller than a first selection threshold in the sleeping video is selected as a first selected frame 10 in step 304. Also, the frame that follows to the first selected frame 10 and has a difference between images of this frame and the first selected frame 10 that is followed by and the closest to this frame not smaller than a second selection threshold in the sleeping video is selected as a second selected frame 20 in step 304.

In step 304, as discussed above, if a second selected frame 20 is selected, frames that are spaced at a predetermined interval from each other from the first selected frame 10 that is followed by and the closest to the second selected frame 20 to the second selected frame 20 are selected as frames that include images to be used to estimate a lying posture. In addition, if the first and second selected frames 10 and 20, and the frames that are spaced at a predetermined interval between the first and second selected frames 10 and 20 are selected, previous and subsequent frames of the first and second selected frames 10 and 20, and the frames that are spaced at a predetermined interval between the first and second selected frames 10 and 20 are selected as frames that include images to be used to estimate a lying posture. After the selection of the frames (after step 304 is completed), the procedure goes to step 305.

In step 305, it is determined whether the moving image ends (all frames in the sleeping video are read). If it is determined that the moving image ends (all frames in the sleeping video are read), the procedure goes to step 306. If it is not determined that the moving image ends (all frames in the sleeping video are read), the procedure goes back to step 301. Steps 301 to 305 are an example of a "selection step" in the claims. In other words, frames in the sleeping video that have a difference between images of the frames not smaller than the threshold (at least a first or second selection threshold) are selected as selected frames corresponding to a lying posture change of the subject 200 in the sleeping video.

In step 306, a shortened video is provided as output. In step 306, the selected frames (first and second selected frames 10 and 20) selected from the sleeping video in step 304 (selection step) are combined so as to generate a shortened video so that the shortened video is provided as output. In this embodiment, as discussed above, the first and second selected frames 10 and 20, the frames that are spaced at a predetermined interval between the first and second selected frames 10 and 20, and the previous and subsequent frames of these frames are used to generate the shortened video. Step 306 is an example of a "moving-image-shortening step" in the claims. After the shortened video is provided (after step 306 is completed), the procedure goes to step 307(FIG. 13).

In step 307, images of the frames in the shortened video are sequentially provided to the lying posture estimation model (see FIG. 9). Subsequently, the procedure goes to step 308.

In step 308, it is determined whether conditions of rotation angle change are satisfied. In this embodiment, it is determined that conditions of rotation angle change are satisfied if a predetermined time elapses in the shortened video or if a difference between images of one frame and a later frame becomes not smaller than a rotation threshold. If it is determined that conditions of rotation angle change are satisfied, the procedure goes to step 309. If it is not determined that conditions of rotation angle change are satisfied, the procedure goes to step 310.

In step 309, an image rotation angle is determined. In step 309, images of frames in the shortened video are rotated by a plurality of angles prior to lying posture estimation, and a rotation angle of an image to be used to estimate the lying posture in the lying posture estimation is determined. In step 309, as discussed above, a lying posture of the subject 200 while sleeping for each of the images rotated to a plurality of angles is estimated. Subsequently, a rotation angle of an image that can provide optimal accuracy of lying posture estimation is determined as the rotation angle based on certainty factors of joint points provided by the lying posture estimation model. Step 309 is an example of a "rotation angle determination step" in the claims. After step 309 is completed, the procedure goes to step 310.

In step 310, the image is rotated. If an image rotation angle is determined (changed) in step 309, the image is rotated to the determined image rotation angle in step 310. If the processing in step 209 is not performed (if none of the predetermined conditions is satisfied in an image of the read frame), the image is rotated to the same rotation angle as a frame that is the closest to this frame and has been determined its rotation angle prior to the lying posture estimation. After image rotation, the procedure goes to step 311.

In step 311, the lying posture estimation is performed. In step 311, a lying posture of the subject 200 while sleeping based on the sleeping video while sleeping is estimated. Step 311 is an example of a "lying posture estimation step" in the claims.

In step 311, a lying posture of the subject 200 while sleeping is estimated by using the lying posture estimation model as discussed above. In this embodiment, as discussed above, a lying posture of the subject 200 while sleeping is estimated by using images of the selected frames (images in the frames of the shortened video), which are selected in step 304 prior to step 311. After step 311 (lying posture estimation) is completed, the procedure goes to step 312.

Subsequently, in step 312, a lying posture estimation result is determined. In step 12, it is determine based on certainty factors of the lying posture estimation acquired by the lying posture estimation model (learned model) in the lying posture estimation in the sleep of the subject 200 and a predetermined validity threshold whether the lying posture estimation result estimated by the lying posture estimation model (learned model) is valid. Step 312 is an example of an "estimation result determination step" in the claims. After step 312 is completed, the procedure goes to steps 313 and 314.

In step 313, a lying posture is classified. In step 313, a lying posture of the subject 200 while sleeping is classified based on the lying posture estimation result in step 311. Note that a lying posture of subject 200 is classified if it is determined that the lying posture estimation result in step 311 (lying posture estimation step) is valid in step 312 (estimation result determination step). Step 313 is an example of a "lying posture estimation step" in the claims.

In addition, in step 314, body movement is measured. In step 314, body movement is measured based on the lying posture estimation result in step 311 (lying posture estimation step). Step 314 is an example of a "body movement measurement step" in the claims. After steps 313 and 314 are completed, the procedure goes to step 315.

In step 315, a display step of displaying at least one of the lying posture classification result and a body movement measurement result. In this embodiment, both the lying posture classification result and the body movement measurement result are displayed on the display 2 based on control by the PC 1. Step 315 is an example of a "display step" in the claims.

Also, in step 315, the lying posture classification results (classification of the lying postures) are displayed if lying postures are classified in the lying posture classification in accordance with determination results of the estimated result determination as discussed above. In addition, in step 315, the lying posture classification results and the body movement measurement results are displayed in a time series (see FIG. 10 and 11).

On the other hand, if a lying posture is not classified in accordance with a determination result of the estimated result determination, an indication that indicates that the lying posture is not classified (see FIG. 10) or an indication indicating the certainty factor of the lying posture estimation (see FIG. 11) is indicated as discussed above.

### Advantages of the Embodiment

In this embodiment, the following advantages are obtained.

According to this embodiment, because the lying posture classification results classified based on the lying posture estimation result(s) are displayed on a display 2, users can see the lying posture classification result and know the classification of the lying posture of a subject 200while sleeping. As a result, dissimilar to a case in which lying posture classification results are not displayed, users can easily grasp the lying posture classification and lying posture changes of the subject 200 in a sleeping video. Also, body movement measurement results measured based on lying posture estimation results are displayed on the display 2, users can see the body movement measurement results and know an amount of body movement change of the subject 200 while sleeping. As a result, dissimilar to a case in which a body movement measurement results are not displayed, users can easily grasp body movement changes of the subject 200 in a sleeping video. Therefore, users can easily grasp lying posture changes (sleeping posture changes) of the subject 200 in a sleeping video from the lying posture classification results and the body movement measurement results.

In addition, the following additional advantages can be obtained by a sleeping video analysis method according to the aforementioned embodiment added with configurations discussed below.

The sleeping video analysis method according to the aforementioned embodiment includes step 304 (selection step) of sequentially reading images of frames in the sleeping video, and selecting frames that have a difference between images of frames not smaller than a selection threshold in the sleeping video as selected frames corresponding to a lying posture change of the subject 200 in the sleeping video. Accordingly, from frames in the sleeping video, frames in which a lying posture of the subject 200 is changed are selected. As a result, users can easily see images in which a lying posture of the subject 200 is changed even in a case of a long sleeping video.

Also, in the sleeping video analysis method according to the aforementioned embodiment, step 311 (lying posture estimation step) includes a step of estimating a lying posture of the subject 200 while sleeping by using an image of the selected frame, which is selected in step 304 (selection step) prior to step 311 (lying posture estimation step). Consequently, as compared with a case in which a lying posture of the subject 200 is estimated from all frames (entire frames) in the sleeping video, the number of lying posture estimation processes in the sleeping video can be reduced.

Also, in the sleeping video analysis method according to the aforementioned embodiment, in step 304 (selection step), in addition to the selected frames (first and second selected frames 10 and 20), frames previous and subsequent to each selected frame are selected as frames that include images to be used to estimate a lying posture in step 311 (lying posture estimation step). Accordingly, dissimilar to a case in which only the selected frames (first and second selected frames 10 and 20) are selected as frames that include images to be used to estimate a lying posture, frames previous and subsequent to each selected frame, which has a large difference between images of frames, can be selected as frames that include images to be used to estimate a lying posture. As a result, based on images of movement of a body of the subject 200 that are captured in the frames previous and subsequent to the frame in which a lying posture of the subject 200 is largely changed, the lying posture can be estimated. Consequently, dissimilar to a case in which only the selected frames (first and second selected frames 10 and 20) are selected as frames that include images to be used to estimate a lying posture, a lying posture change of the subject 200 can be accurately grasped.

Also, in the sleeping video analysis method according to the aforementioned embodiment, in steps 301 to 305 (selection step), images of frames in the sleeping video are sequentially read, and a frame that has a difference between images of frames adjacent to each other not smaller than a first selection threshold in the sleeping video is selected as a first selected frame 10. In addition, the frame that follows to the first selected frame 10 and has a difference between images of this frame and the first selected frame 10 that is followed by and the closest to this frame not smaller than a second selection threshold in the sleeping video is selected as a second selected frame 20 in step 304 (selection step). Accordingly, dissimilar to a case in which a frame is selected based on only a difference between images of frames adjacent to each other, even if a lying posture of the subject 200 is slowly changed over a plurality of frames,

Also, in the sleeping video analysis method according to the aforementioned embodiment, in step 304 (selection step), frames that are spaced at a predetermined interval from each other from the first selected frame 10 that is followed by and the closest to the second selected frame 20 to the second selected frame 20 are selected as frames that include images to be used to estimate a lying posture in step 311 (lying posture estimation) in addition to the first and second selected frames 10 and 20. Accordingly, frames from the first selected frame 10 to the second selected frame 20 can be used to estimate the lying posture so that lying posture change can be estimated in stages from the first selected frame 10 to the second selected frame 20. As a result, users can easily grasp the lying posture (sleeping posture) change of the subject 200 from the first selected frame 10 to the second selected frame 20.

Also, in the sleeping video analysis method according to the aforementioned embodiment, in step 315 (display step), the lying posture classification results and the body movement measurement results are displayed in a time series. According to this configuration in which the lying posture classification results and the body movement measurement results are displayed in a time series, users can easily see lying posture changes and body movement changes in a time series in sleeping posture and body motion of the subject 200 in a time series.

Also, in the sleeping video analysis method according to the aforementioned embodiment includes step 306 (video-shortening step) of combining the selected frames selected from the sleeping video in step 304 (selection step) so as to generate a shortened video. Accordingly, it is possible to generate a video that includes images in a time period in which a lying posture of the subject 200 is changed extracted from the sleeping video. As a result, as compared with a case in which users see the entire sleeping video of the subject 200, users can efficiently see lying posture changes from the shortened video.

Also, the sleeping video analysis method according to the aforementioned embodiment includes step 309 (rotation angle determination step) of rotating images of frames in the shortened video by a plurality of angles prior to step 311 (lying posture estimation step) to estimate a lying posture the subject 200 while sleeping for each of the images rotated to a plurality of angles, and selectively determining a rotation angle of an image to be used to estimate the lying posture in step 311 (lying posture estimation step) from the images rotated. Because an image that can provide optimal accuracy of lying posture estimation can be selected from images that are rotated to a plurality of rotation angles and used to estimate a lying posture, the lying posture can be accurately estimated.

Also, in the sleeping video analysis method according to the aforementioned embodiment, in step 309 (rotation angle determination step), images of frames in the shortened video are rotated by a plurality of angles and a rotation angle of an image to be used to estimate the lying posture in step 311 (lying posture estimation step) is selectively determined from the images rotated at the timing of every time a predetermined time elapses in the shortened video and every time a difference between images of one frame and a previous frame in the shortened video becomes not smaller a rotation threshold. Accordingly, as compared with a case in which a rotation angle of an image to be used to estimate the lying posture is determined for images of all frames (entire frames) in the shortened video, the number of determination processes of determining a rotation angle of an image to be used to estimate the lying posture in step 311 (lying posture estimation step) can be reduced. Also, because a rotation angle of an image to be used to estimate the lying posture is determined at the timing of every time a predetermined time elapses in the shortened video and every time a difference between images of one frame and a later frame in the shortened video becomes not smaller a rotation threshold, the lying posture can be accurately estimated as compared with a case in which a rotation angle of an image to be used to estimate the lying posture in step 311 (lying posture estimation step) is selectively determined from the images rotated at the timing of only one of every time a predetermined time elapses in the shortened video and every time a difference between images of one frame and a previous frame in the shortened video becomes not smaller a rotation threshold.

Also, the sleeping video analysis method according to the aforementioned embodiment includes step 312 (estimated result determination step) of determining whether lying posture estimation results estimated by the lying posture estimation model (learned model) are valid based on a certainty factor of the lying posture estimation acquired by the learned model in the lying posture estimation of the subject 200 while sleeping and a predetermined validity threshold. Accordingly, because it is determined whether lying posture estimation results estimated by the learned model are valid, it is possible to easily determine whether lying posture estimation results have sufficient accuracy to use the lying posture estimation results for lying posture classification and body movement measurement.

Also, in the sleeping video analysis method according to the aforementioned embodiment, in step 313 (lying posture classification step), a lying posture of the subject 200 is classified if it is determined that the lying posture estimation result in step 311 (lying posture estimation step) is valid in step 312 (estimation result determination step). In addition, in step 315 (display step), the lying posture classification results in step 313 (lying posture classification step) is displayed if the lying posture is classified in step 313 (lying posture classification step) in accordance with a determination result in step 312 (estimated result determination step). Accordingly, users can easily see the classification of lying postures of the subject 200 from the displayed lying posture classification results. In addition, in step 315 (display step), at least one of an indication indicating that no classification is made and an indication indicating the certainty factor of the lying posture estimation acquired by the lying posture estimation model (learned model) can be selected and displayed if the lying posture is not classified in step 313 (lying posture classification step) in accordance with the determination result in step 312 (estimated result determination step). Accordingly, in a case in which an indication indicating that no classification is made is displayed, users can easily know that the lying posture is not classified in step 313 (lying posture classification step). Also, in a case in which an indication indicating the certainty factor of the lying posture estimation acquired by the lying posture estimation model (learned model) is displayed, users can easily know a low certainty factor of the lying posture estimation acquired by the lying posture estimation model, which means that no classification is made.

### Modified Embodiment

Note that the embodiment disclosed this time must be considered as illustrative in all points and not restrictive. The scope of the present invention is not shown by the above description of the embodiments but by the scope of claims for patent, and all modifications (modified examples) within the meaning and scope equivalent to the scope of claims for patent are further included.

While the example in which ZNCC (Zero-Mean Normalized Cross Correlation) is used as a technique that calculates a difference between frames in the sleeping video to select a frame has been shown in the aforementioned embodiment, the present invention is not limited to this. In the present invention, a technique that acquires a correlation between images of frames or pixel differences between frames may be used as the technique, which calculates a difference between frames in the sleeping video. For example, using a technique such as SSD (Sum of Squared difference), SAD (Sum of Absolute difference) or NCC (Normalized Cross Correlation), a frame (first or second selected frame) may be selected on a condition that a calculated value is not greater (or not smaller) than a predetermined threshold as a determination that a difference between images is not smaller than a first threshold. Also, a plurality of techniques may be used for the frame selection.

While the example in which both a set of lying posture classification results and a set of body movement measurement results are displayed has been shown in the aforementioned embodiment, the present invention is not limited to this. In the present invention, one of the lying posture classification result set and the body movement measurement result set may be selected and displayed. Alternatively, one of the lying posture classification result set and the body movement measurement result set may be only displayed. For example, in a display step, only the lying posture classification result set may be displayed. In this case, users can easily grasp the lying posture changes of the subject in the sleeping video from the displayed classification result set of the lying posture of the subject while sleeping. Alternatively, in a display step, only the body movement measurement result set may be displayed. In this case, users can easily grasp the body movement changes of the subject in the sleeping video from the displayed body movement measurement result set of the subject while sleeping.

While the example in which a lying posture of the subject 200 in the sleep is estimated by using an image of a frame (an image in the shortened video) that is selected prior to lying posture estimation has been shown in the aforementioned embodiment, the present invention is not limited to this. In the present invention, a lying posture of the subject while sleeping may be estimated by using images of all frames (entire frames) in the sleeping video.

Also, while the example in which, in addition to the selected frame (a frame that has a difference between images of frames not smaller than a selection threshold in the sleeping video), frames previous and subsequent to the selected frame are selected as frames that include images to be used to estimate a lying posture in the lying posture estimation step has been shown in the aforementioned embodiment, the present invention is not limited to this. In the present invention, only an image of the selected frame (the frame that has a difference between images of frames not smaller than a selection threshold in the sleeping video) may be used to estimate a lying posture in the lying posture estimation step.

Also, while the example in which the frame that follows to the first selected frame 10 and has a difference between images of this frame and the first selected frame 10 that is followed by and the closest to this frame not smaller than a second selection threshold in the sleeping video is selected as a second selected frame 20 has been shown in the aforementioned embodiment, the present invention is not limited to this. In the present invention, only the first selected frame may be selected as a frame corresponding to an image used to estimate a lying posture.

Also, while the example in which frames that are spaced at a predetermined interval from each other from the first selected frame 10 that is followed by and the closest to the second selected frame 20 to the second selected frame 20 are selected as frames that include images to be used to estimate a lying posture in step 311 (lying posture estimation step) in addition to the first and second selected frames 10 and 20 has been shown in the aforementioned embodiment, the present invention is not limited to this. In the present invention, only the first and second selected frames may be selected as frames corresponding to images used to estimate a lying posture. Also, while the example in which images of frames in the sleeping video are sequentially read and a frame that has a difference between images of frames adjacent to each other not smaller than a first selection threshold in the sleeping video is selected as a first selected frame 10 has been shown in the aforementioned embodiment, the present invention is not limited to this. In the present invention, frames used to calculate a difference between their corresponding images are not necessarily limited to frames adjacent to each other but frames that are spaced at a predetermined interval from each other may be picked up to calculate a difference between their corresponding images so that the selected frame (the frame that has a difference between images of frames not smaller than a selection threshold in the sleeping video) may be selected.

Also, while the example in which the selected frames selected from the sleeping video are combined so as to generate a shortened video has been shown in the aforementioned embodiment, the present invention is not limited to this. In the present invention, parts (frame number or time range) of the sleeping video corresponding to the selected frames in which a lying posture of the subject is changed may be simply displayed to indicate correspondence to the sleeping video. In this case, a result (lying posture estimation result, lying posture classification result or body movement measurement result) in a selected frame may be displayed in response to a user instruction that selects a part (frame number or time range) of the sleeping video corresponding to the selected frame. Also, in the present invention, the lying posture classification result or body movement measurement result of the frame in the shortened video may be displayed from its corresponding time in the sleeping video (long time video) in accordance with the aforementioned correspondence between the sleeping video (long time video) and the shortened video (see paragraph [0032]). In the original sleeping video (long time video), if a frame has neither lying posture classification result nor body movement measurement result, and a previous frame that is followed by and the closest to this frame has some result, the same result (lying posture classification result or body movement measurement result) as the previous frame can be displayed for this frame, for example. Alternatively, from frames that have some result and are forward of and backward of this frame in time sequence, a closest frame that is the closest to this frame in time sequence may be selected to display its result for this frame.

Also, while the example in which an image in each frame in the shortened video is rotated prior to step 311 (lying posture estimation step) has been shown in the aforementioned embodiment, the present invention is not limited to this. In the present invention, a lying posture may be estimated without rotating an image used to estimate the lying posture prior to the lying posture estimation.

Also, while the example in which in the rotation angle determination step, images of frames in the shortened video are rotated by a plurality of angles and a rotation angle of an image to be used to estimate the lying posture in the lying posture estimation step is selectively determined from the images rotated at the timing of every time a predetermined time elapses in the shortened video and every time a difference between images of one frame and a previous frame in the shortened video becomes not smaller a rotation threshold has been shown in the aforementioned embodiment, the present invention is not limited to this. In the present invention, in the rotation angle determination step, images of all frames (entire frames) in the shortened video may be rotated by a plurality of angles, and an image rotation angle of each image to be used to estimate the lying posture in the lying posture estimation step may be determined.

Also, while the example in which images of frames in the shortened video are rotated by a plurality of angles and a rotation angle of an image to be used to estimate the lying posture in step 311 (lying posture estimation step) is selectively determined from the images rotated at the timing of every time a predetermined time elapses in the shortened video and every time a difference between images of one frame and a previous frame in the shortened video becomes not smaller a rotation threshold has been shown in the aforementioned embodiment, the present invention is not limited to this. In the present invention, an image rotation angle to be used to estimate a lying posture may be determined at the timing at least one of every time a predetermined time elapses in the shortened video and every time a difference between images of one frame and a later frame in the shortened video becomes not smaller a rotation threshold. For example, an image rotation angle to be used to estimate a lying posture may be determined every time the predetermined time elapses in the shortened video. Accordingly, as compared with a case in which a rotation angle of an image to be used to estimate the lying posture is determined for images of all frames (entire frames) in the shortened video, the number of determination processes of determining a rotation angle of an image to be used to estimate the lying posture can be reduced. Also, an image rotation angle to be used to estimate a lying posture may be determined every time a difference between images of one frame and a later frame in the shortened video becomes not smaller a rotation threshold. Also, in this case, as compared with a case in which a rotation angle of an image to be used to estimate the lying posture is determined for images of all frames (entire frames) in the shortened video, the number of determination processes of determining a rotation angle of an image to be used to estimate the lying posture can be reduced.

Also, while the example in which step 312 (estimated result determination step) of determining whether lying posture estimation results estimated by the learned model are valid based on a certainty factor of the lying posture estimation acquired by the learned model in the lying posture estimation of the subject 200 while sleeping and a predetermined validity threshold prior to step 313 (lying posture classification step) is provided has been shown in the aforementioned embodiment, the present invention is not limited to this. In the present invention, the lying posture classification in the lying posture classification step or the body movement measurement in the body movement measurement step may performed without determining whether the lying posture estimation result by the learned model is valid.

Also, while the example in which an indication indicating that no classification is made and an indication indicating the certainty factor of the lying posture estimation acquired by the lying posture estimation model (learned model) are selected and displayed has been shown in the aforementioned embodiment, the present invention is not limited to this. In the present invention, if a lying posture is not classified in the lying posture classification in accordance with a determination result in the estimated result determination step, only an indication that indicates that the lying posture is not classified may be displayed. In the present invention, if a lying posture is not classified in the lying posture classification in accordance with a determination result in the estimated result determination step, only an indication indicating the certainty factor of the lying posture estimation acquired by the learned model may be displayed.

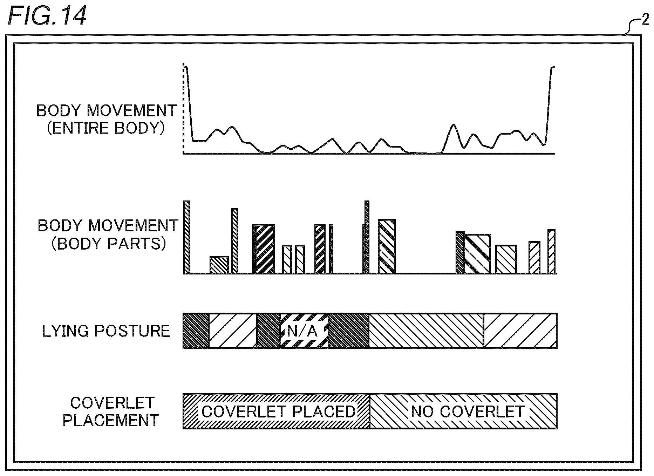

Also, while the example in which a set of lying posture classification results and a set of body movement measurement results are displayed has been shown in the aforementioned embodiment, the present invention is not limited to this. In the present invention, as shown in FIG. 14, in addition to the lying posture classification results and the body movement measurement results, placement of a coverlet (whether a coverlet is placed on the subject) may be displayed.

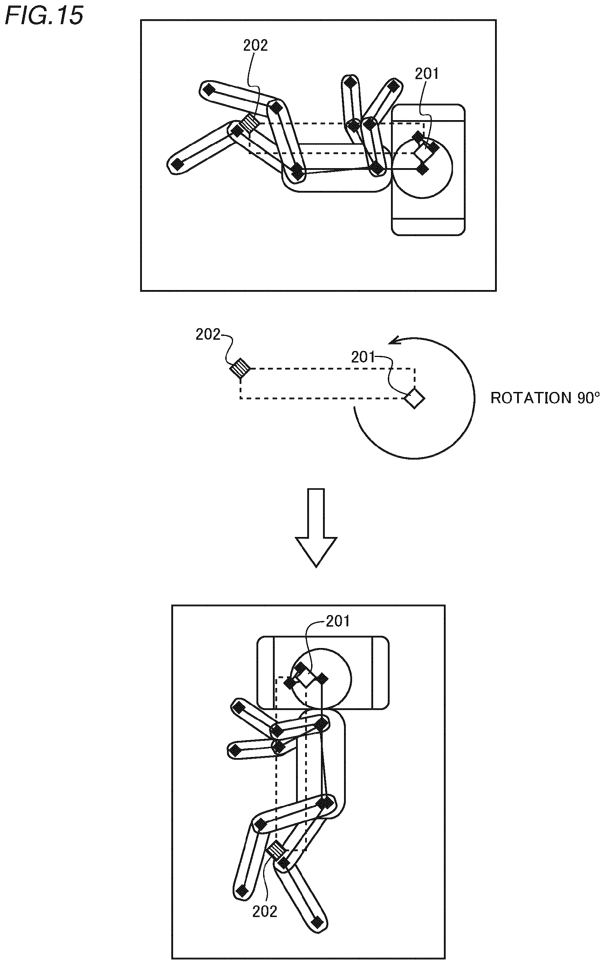

Also, while the example in which a rotation angle of an image that provides the highest average value of certainty factors of joint points provided by the lying posture estimation model (learned model) is selected as a rotation angle of an image to be used to estimate the lying posture prior to lying posture estimation has been shown in the aforementioned embodiment, the present invention is not limited to this. In the present invention, prior to lying posture estimation, frames in the sleeping video or the shortened video may be sequentially analyzed so that a body orientation of the subject is calculated based on results of the lying posture estimation, etc., and if the body orientation of the subject is horizontal or upside down an image may be rotated so as to place the body orientation of the subject at a proper angle (an angle that places the head of the subject on the upside) in a frame subjected to the calculation or a frame subsequent to this frame. For example, as shown in FIG. 15, a center point 201 of the head-side joint points (eyes, ears, nose, etc.) and a center point 202 of the leg-side joint points (waist, knees, ankles, etc.) are calculated, and an image is rotated to an angle that places the center point 201 of the head-side joint points on the upside with respect to the center point 202 of the leg-side joint points. In this case, such a rotation angle may be selected from angles staggered 90° or specified to any angle that places the head and the legs in vertical positions.

Also, while the example in which the procedure of a sleeping video analysis method according to the present invention has been described by using a flow-driven type flowchart in which processes are sequentially executed along a processing flow for sake of illustration in the aforementioned embodiment, the present invention is not limited to this. In the present invention, the procedure of a sleeping video analysis method may be realized by an event-driven type processing that executes processes in response to occurrence of events. In this case, the procedure of a sleeping video analysis method may be realized entirely by such an event-driven type processing or by a combination of such a flow-driven type processing and such an event-driven type processing.

### Modes

The aforementioned exemplary embodiment will be understood as concrete examples of the following modes by those skilled in the art.

### (Mode Item 1)

A sleeping video analysis method includes a lying posture estimation step of estimating a lying posture of the subject while sleeping based on a sleeping video of the subject while sleeping; a lying posture classification step of classifying the lying posture of the subject while sleeping based on a lying posture estimation result(s) of the lying posture estimation step; a body movement measurement step of measuring body movement of the subject while sleeping based on the lying posture estimation result(s) of the lying posture estimation step; and a display step of displaying at least one of a lying posture classification result(s) of the lying posture classification step and a body movement measurement result(s) of the body movement measurement step.

### (Mode Item 2)

The sleeping video analysis method according to mode item 1 further includes a selection step of sequentially reading images of frames in the sleeping video, and selecting a frame(s) that has/have a difference between images of frames not smaller than a selection threshold in the sleeping video as a selected frame(s) corresponding to a lying posture change of the subject in the sleeping video.

### (Mode Item 3)

The sleeping video analysis method according to mode item 2, wherein the lying posture estimation step includes a step of estimating a lying posture of the subject while sleeping by using an image of the selected frame, which is selected in the selection step prior to the lying posture estimation step.

### (Mode Item 4)

The sleeping video analysis method according to mode item 3, wherein the selection step includes a step of additionally selecting, in addition to the selected frame, frames previous and subsequent to the selected frame as frames that include images to be used to estimate a lying posture in the lying posture estimation step.

### (Mode Item 5)

The sleeping video analysis method according to any of mode items 2 to 4, wherein the selection step includes a step of sequentially reading images of frames in the sleeping video and selecting a frame(s) that has/have a difference between images of frames adjacent to each other not smaller than a first selection threshold in the sleeping video as a first selected frame(s), and a step of selecting a frame that has a difference between images of this frame and the first selected frame that is followed by and the closest to this frame not smaller than a second selection threshold in the sleeping video as a second selected frame.

### (Mode Item 6)

The sleeping video analysis method according to mode item 5, wherein the selection step further includes a step of selecting, in addition to the first and second selected frames, frames that are spaced at a predetermined interval from each other from the first selected frame that is followed by and the closest to the second selected frame to the second selected frame as frames that include images to be used to estimate a lying posture in the lying posture estimation step.

### (Mode Item 7)

The sleeping video analysis method according to any of mode items 1 to 6, wherein the display step includes a step of displaying the lying posture classification results of the lying posture classification step and the body movement measurement results of the body movement measurement step in a time series.

### (Mode Item 8)

The sleeping video analysis method according to any of mode items 2 to 6 further includes a moving-image-shortening step of combining the selected frames selected from the sleeping video in the selection step so as to generate a shortened video.

### (Mode Item 9)

The sleeping video analysis method according to mode item 8 further includes a rotation angle determination step of rotating images of frames in the shortened video by a plurality of angles prior to the lying posture estimation step to estimate a lying posture of the subject while sleeping is estimated for the images rotated to a plurality of angles, and determining a rotation angle of an image to be used to estimate the lying posture in the lying posture estimation step.

### (Mode Item 10)

The sleeping video analysis method according to mode item 9 further includes the rotation angle determination step includes rotating images of frames in the shortened video by a plurality of angles and determining a rotation angle of an image to be used to estimate the lying posture in the lying posture estimation step at the timing of at least one of every time a predetermined time elapses in the shortened video and every time a difference between images of one frame and a previous frame in the shortened video becomes not smaller than a rotation threshold.

### (Mode Item 11)

The sleeping video analysis method according to any of mode items 1 to 10, wherein the lying posture estimation step includes a step of estimating the lying posture of the subject while sleeping based on a learned model produced by machine learning; and the method further includes an estimated result determination step of determining whether the lying posture estimation result(s) estimated by the learned model is/are valid based on a certainty factor of the lying posture estimation acquired by the learned model in the lying posture estimation in the sleep of the subject and a predetermined validity threshold.

### (Mode Item 12)

The sleeping video analysis method according to mode item 11, wherein the lying posture classification step classifies the lying posture of the subject if it is determined that the lying posture estimation result in the lying posture estimation step is valid in the estimated result determination step; and the display step includes a step of displaying the lying posture classification result(s) of the lying posture classification step if the lying posture has been classified in the lying posture classification step in accordance with a determination result of the estimated result determination step, and a step of displaying at least one of an indication indicating that no classification is made and an indication indicating the certainty factor of the lying posture estimation acquired by the learned model if the lying posture is not classified in the lying posture classification step in accordance with the determination result of the estimated result determination step.

### (Mode Item 13)

A program which executes a computer to perform the sleeping video analysis method according to according to any of mode items 1 to 12.

### (Mode Item 14)

A computer-readable storage medium having the program according to mode item 13.

### (Mode Item 15)

A sleeping video analysis apparatus includes a controller configured to analyze a sleeping video, which is a moving image of a subject during sleep; and a display configured to display an analysis result(s) analyzed by the controller, wherein the controller is configured to control a lying posture estimation function of estimating a lying posture of the subject while sleeping based on the sleeping video, a lying posture classification function of classifying the lying posture of the subject while sleeping based on a lying posture estimation result(s) in the lying posture estimation function, a body movement measurement function of measuring body movement of the subject while sleeping based on the lying posture estimation result(s) of the lying posture estimation function, and a display function of displaying at least one of a lying posture classification result(s) of the lying posture classification function and a body movement measurement result(s) of the body movement measurement function.

### (Mode Item 16)

The sleeping video analysis apparatus according to mode item 15, wherein the controller is configured to control a function of sequentially reading images of frames in the sleeping video, and selecting a frame(s) that has/have a difference between images of frames not smaller than a selection threshold in the sleeping video as a selected frame(s) corresponding to a lying posture change of the subject in the sleeping video.

### Description of Reference Numerals

1: PC (controller)
2: display device (display)
10: first selected frames
20: second selected frames
100: analysis apparatuses (sleeping video analysis apparatus)
200: subject

## Claims

1. A sleeping video analysis method comprising:
a lying posture estimation step of estimating a lying posture of a subject while sleeping based on a sleeping video of the subject while sleeping;
a lying posture classification step of classifying the lying posture of the subject while sleeping based on a lying posture estimation result(s) of the lying posture estimation step;
a body movement measurement step of measuring body movement of the subject while sleeping based on the lying posture estimation result(s) of the lying posture estimation step; and
a display step of displaying at least one of a lying posture classification result(s) of the lying posture classification step and a body movement measurement result(s) of the body movement measurement step.

2. The sleeping video analysis method according to claim 1 further comprising
a selection step of sequentially reading images of frames in the sleeping video, and selecting a frame(s) that has/have a difference between images of frames not smaller than a selection threshold in the sleeping video as a selected frame(s) corresponding to a lying posture change of the subject in the sleeping video.

3. The sleeping video analysis method according to claim 2, wherein the lying posture estimation step includes a step of estimating a lying posture of the subject while sleeping by using an image of the selected frame, which is selected in the selection step prior to the lying posture estimation step.

4. The sleeping video analysis method according to claim 3, wherein the selection step includes a step of additionally selecting, in addition to the selected frame, frames previous and subsequent to the selected frame as frames that include images to be used to estimate a lying posture in the lying posture estimation step.

5. The sleeping video analysis method according to claim 2, wherein
the selection step includes
a step of sequentially reading images of frames in the sleeping video and selecting a frame(s) that has/have a difference between images of frames adjacent to each other not smaller than a first selection threshold in the sleeping video as a first selected frame(s), and
a step of selecting a frame that has a difference between images of this frame and the first selected frame that is followed by and the closest to this frame not smaller than a second selection threshold in the sleeping video as a second selected frame.

6. The sleeping video analysis method according to claim 5, wherein the selection step further includes a step of selecting, in addition to the first and second selected frames, frames that are spaced at a predetermined interval from each other from the first selected frame that is followed by and the closest to the second selected frame to the second selected frame as frames that include images to be used to estimate a lying posture in the lying posture estimation step.

7. The sleeping video analysis method according to claim 1, wherein the display step includes a step of displaying the lying posture classification results of the lying posture classification step and the body movement measurement results of the body movement measurement step in a time series.

8. The sleeping video analysis method according to claim 2 further comprising a video-shortening step of combining the selected frames selected from the sleeping video in the selection step so as to generate a shortened video.

9. The sleeping video analysis method according to claim 8 further comprising a rotation angle determination step of rotating images of frames in the shortened video by a plurality of angles prior to the lying posture estimation step to estimate a lying posture of the subject while sleeping for each of the images rotated to a plurality of angles, and determining a rotation angle of an image to be used to estimate the lying posture in the lying posture estimation step.

10. The sleeping video analysis method according to claim 9, wherein the rotation angle determination step includes rotating images of frames in the shortened video by a plurality of angles and determining a rotation angle of an image to be used to estimate the lying posture in the lying posture estimation step at the timing of at least one of every time a predetermined time elapses in the shortened video and every time a difference between images of one frame and a previous frame in the shortened video becomes not smaller than a rotation threshold.

11. The sleeping video analysis method according to claim 1, wherein
the lying posture estimation step includes a step of estimating the lying posture of the subject while sleeping based on a learned model produced by machine learning; and
the method further includes an estimated result determination step of determining whether the lying posture estimation result(s) estimated by the learned model is/are valid based on a certainty factor of the lying posture estimation acquired by the learned model in the lying posture estimation of the subject while sleeping and a predetermined validity threshold.

12. The sleeping video analysis method according to claim 11, wherein
the lying posture classification step classifies the lying posture of the subject if it is determined that the lying posture estimation result in the lying posture estimation step is valid in the estimated result determination step; and
the display step includes
a step of displaying the lying posture classification result(s) of the lying posture classification step if the lying posture has been classified in the lying posture classification step in accordance with a determination result of the estimated result determination step, and
a step of displaying at least one of an indication indicating that no classification is made and an indication indicating the certainty factor of the lying posture estimation acquired by the learned model if the lying posture is not classified in the lying posture classification step in accordance with the determination result of the estimated result determination step.

13. A program which executes a computer to perform the sleeping video analysis method according to claim 1.

14. A computer-readable storage medium having the program according to claim 13.

15. A sleeping video analysis apparatus comprising:
a controller configured to analyze a sleeping video of a subject while sleeping; and
a display configured to display an analysis result(s) analyzed by the controller, wherein
the controller is configured to control
a lying posture estimation function of estimating a lying posture of the subject while sleeping based on the sleeping video,
a lying posture classification function of classifying the lying posture of the subject while sleeping based on a lying posture estimation result(s) in the lying posture estimation function,
a body movement measurement function of measuring body movement of the subject while sleeping based on the lying posture estimation result(s) of the lying posture estimation function, and
a display function of displaying at least one of a lying posture classification result(s) of the lying posture classification function and a body movement measurement result(s) of the body movement measurement function.

16. The sleeping video analysis apparatus according to claim 15, wherein the controller is configured to control a function of sequentially reading images of frames in the sleeping video, and selecting a frame(s) that has/have a difference between images of frames not smaller than a selection threshold in the sleeping video as a selected frame(s) corresponding to a lying posture change of the subject in the sleeping video.
